# FASCICULE DE BREVET EUROPEEN

(11) **EP 0 752 004 B1**
(45) Date de publication et mention de la délivrance du brevet: **09.08.2006**
(21) Numéro de dépôt: 95914402.3
(22) Date de dépôt: 23.03.1995
(51) Int. Cl.: C12N 15/86, A61K 48/00, C12N 15/12, C07K 14/475, C12N 7/01, C12N 5/10, A61K 39/235

(54) **ADENOVIRUS RECOMBINANTS CODANT POUR LE FACTEUR NEUROTROPHIQUE DES CELLULES GLIALES (GDNF)**
FÜR GDNF-KODIERENDE REKOMBINANTE ADENOVIREN
RECOMBINANT ADENOVIRUSES CODING FOR GLIAL-DERIVED NEUROTROPHIC FACTOR (GDNF)

(30) Priorité: 25.03.1994 FR 9403542
(43) Date de publication de la demande: 08.01.1997
(73) Titulaire: Aventis Pharma S.A., 92160 Antony (FR)
(72) Inventeur: HORELLOU, Philippe, F-75014 Paris (FR); MALLET, Jacques, F-75013 Paris (FR); PERRICAUDET, Michel, F-28320 Ecrosnes (FR); REVAH, Frédéric, F-92160 Antony (FR); VIGNE, Emmanuelle, F-94200 Ivry-sur-Seine (FR)
(74) Mandataire: Gallois, Valérie
(86) Numéro de dépôt international: PCT/FR1995/000356
(87) Numéro de publication internationale: WO 1995/026408

(56) Documents cités:
- WO-A-94/08026
- WO-A-94/20146
- FR-A- 2 688 514
- SCIENCE, vol.259, 12 Février 1993, LANCASTER, PA, US; pages 988 - 990 LE GAL LA SALLE, G. ET AL.: 'An adenovirus vector for gene transfer into neurons and glia in the brain'
- NOUVELLE REVUE FRANCAISE D'HEMATOLOGIE, vol.35, no.3, 1993 pages 299 - 300 PESCHANSKI, M. ET AL.: 'Transfert de gènes à but thérapeutique dans le système nerveux central' & XIITH CONGRES DE LA SOCIETE FRANCAISE D'HEMATOLOGIE, Juin 1993, STRASBOURG, FR;
- NEUROREPORT, vol.5, no.7, 21 Mars 1994 pages 801 - 804 RIDOUX, V. ET AL.: 'The use of adenovirus vectors for intracerebral grafting of transfected nervous cells'
- MOLECULAR BIOLOGY OF THE CELL, vol.4, no.SUPP, Octobre 1993 page 442A BAETGE, E.E. ET AL.: 'Delivery of a putative Parkinson's factor (GDNF) into the rat CNS using a polymer-encapsulated cell line'
- EUROPEAN JOURNAL OF BIOCHEMISTRY, vol.208, no.2, Septembre 1992, AMSTERDAM, NL; pages 211 - 225 ROEMER, K. ET FRIEDMANN, T.: 'Concepts and strategies for human gene therapy'
- BRAIN RESEARCH, vol.648, no.1, 1994 pages 171 - 175 RIDOUX, V. ET AL.: 'Adenoviral vectors as functional retrograde neuronal tracers'
- MEDECINE / SCIENCES, vol.9, no.2, Février 1993 pages 208 - 210 DANOS, O. ET AL.: 'Réimplantation de cellules génétiquement modifiées dans des néo-organes vascularisés'
- SCIENCE, vol.256, no.5063, 12 Juin 1992, LANCASTER, PA, US; pages 1550 - 1552 CULVER, K.W. ET AL.: 'In vivo gene transfer with retroviral vector-producer cells for treatment of experimental brain tumors'

## Description

La présente invention décrit des adénovirus recombinants comportant une séquence d'ADN codant pour le facteur neurotrophique dérivé des cellules Gliales. L'invention décrit également la préparation de ces vecteurs, les compositions pharmaceutiques les contenant et leur utilisation thérapeutique, notamment en thérapie génique pour le traitement et/ou la prévention de maladies neurodégénératives.

L'augmentation de la durée de vie dans les pays occidentaux s'accompagne d'une croissance régulière des maladies neurodégénératives de type maladie d'Alzheimer, maladie de Parkinson, chorée de Huntington, sclérose latérale amyotrophique, etc. C'est ainsi que la maladie de Parkinson, par exemple, atteint 4% des personnes agées de plus de 65 ans, et la maladie d'Alzheimer atteint 10% des plus de 70 ans et 30% des plus de 80 ans. De manière générale, toutes ces maladies résultent d'une perte progressive de cellules neuronales dans le système nerveux central, voire au sein de structures très localisées comme dans le cas de la maladie de Parkinson.

Au cours de ces dernières années, de nombreuses recherches ont été développées en vu de comprendre les mécanismes de ces dégénérescences liés aux vieillissement dans la perspective de mettre au point des moyens de traitement mais également des moyens de prévention, par la thérapie génique.

En effet, les maladies neurodégénératives traduisant une mort progressive des cellules neuronales, la stimulation de la production des facteurs de croissances, impliqués dans le développement de ces cellules neuronales, est apparue comme une voie possible pour prévenir et/ou s'opposer à cette dégénérescence.

La présente invention a notamment pour objectif de proposer des vecteurs permettant de promouvoir directement la survie des cellules neuronales, impliquées dans ces pathologies, par l'expression efficace et localisée de certains facteurs trophiques.

Les facteurs trophiques sont une classe de molécules ayant des propriétés de stimulation de la croissance neuritique ou de la survie des cellules nerveuses. Le premier facteur possédant des propriétés neurotrophiques, le NGF ("Nerve Growth Factor"), a été caractérisé il y a une quarantaine d'années (pour revue, voir Levi-Montalcini et Angelleti, PhysioL Rev. 48 (1968) 534). Ce n'est que récemment que d'autres facteurs neurotrophiques ont été identifiés, et notamment le facteur neurotrophique dérivé des cellules Gliales (GDNF) (L.-F. Lin, D. Doherty, J. Lile, S. Besktesh, F. Collins, Science, 260,1130-1132 (1993)). Le GDNF est une protéine de 134 acides aminés et de poids moléculaire de 16 kD. Il a pour fonction essentielle de promouvoir in vitro la survie des neurones dopaminergiques.
La présente invention est particulièrement avantageuse pour l'application de GDNF à titre d'agent thérapeutique.
Plus précisément, la présente invention vise la mise au point de vecteurs particulièrement efficaces pour délivrer in vivo et de manière localisée, des quantités thérapeutiquement actives du gène spécifique codant pour le GDNF dans le système nerveux.
Dans la demande copendante n° PCT/EP93/02519, il a été montré que les adénovirus pouvaient être utilisés comme vecteur pour le transfert d'un gène étranger in vivo dans le système nerveux et l'expression de la protéine correspondante.
La présente invention décrit plus particulièrement des constructions nouvelles, particulièrement adaptées et efficaces pour le transfert de facteur neurotrophique dérivé des cellules Gliales (GDNF).
Plus précisément, elle décrit un adénovirus recombinant comprenant une séquence d'ADN codant pour le GDNF ou un de ses dérivés, sa préparation, et son utilisation pour le traitement et/ou la prévention de l'ALS

La demanderesse a ainsi mis en évidence qu'il est possible de construire des adénovirus recombinants contenant une séquence codant pour le GDNF, d'administrer ces adénovirus recombinants in vivo, et que cette administration permet une expression stable et localisée de quantités thérapeutiquement actives de GDNF in vivo, et en particulier dans le système nerveux, et sans effet cytopathologique.

Un premier objet de l'invention réside donc dans l'ulilisation d'un adénovirus recombinant défectif dépourvu des régions de son génome qui sont nécessaires à sa replication autonome dans une cellule cible et comprenant au moins une séquence d'ADN codant pour tout ou une partie active du facteur neurotrophique dérivé des cellules Gliales (GDNF) ou d'un de ses dérivés placée sous le contrôle de signaux permettant son expression dans les cellules nerveuses pour la préparation d'une composition pharmaceutique destinée au traitement et/ou à la prévention de l'ALS,, (sclérose latérale amyotrophique).

Le facteur neurotrophique dérivé des cellules Gliales (GDNF) produit dans le cadre de la présente invention peut être le GDNF humain ou un GDNF animal.

Les séquences d'ADNc codant pour le GDNF humain et le GDNF du rat ont été clonées et séquencées (L.-F. Lin, D. Doherty, J. Lile, S. Besktesh, F. Collins, Science, 260,1130-1132 (1993)).

La séquence d'ADN codant pour le GDNF, utilisée dans le cadre de la présente invention peut être un ADNc, un ADN génomique (ADNg), ou une construction hybride consistant par exemple en un ADNc dans lequel seraient insérés un ou plusieurs introns. Il peut également s'agir de séquences synthétiques ou semisynthétiques. De manière particulièrement avantageuse, la séquence de la présente invention code pour le GDNF précédé de la région pro native (pro GDNF).
De manière particulièrement avantageuse, on utilise un ADNc ou un ADNg. Selon un mode préféré de l'invention, il s'agit une séquence d'ADNg codant pour le GDNF. Son -utilisation peut permettre une meilleure expression dans les cellules humaines.

Bien entendu, préalablement à son incorporation dans un vecteur adénovirus selon l'invention, la séquence d'ADN est avantageusement modifiée, par exemple par mutagénèse dirigée, en particulier pour l'insertion de sites de restriction appropriés. Les séquences décrites dans l'art antérieur ne sont en effet pas construites pour une utilisation selon l'invention, et des adaptations préalables peuvent s'avérer nécessaires, pour obtenir des expressions importantes.

Au sens de la présente invention, on entend par dérivé du GDNF, toute séquence obtenue par modification et codant pour un produit conservant l'une au moins des propriétés biologiques du GDNF (effet trophique et/ou différentiateur). Par modification, on doit entendre toute mutation, substitution, délétion, addition ou modification de nature génétique et/ou chimique. Ces modifications peuvent être réalisées par les techniques connues de l'homme du métier (voir techniques générales de biologie moléculaire ci-après). Les dérivés au sens de l'invention peuvent également être obtenus par hybridation à partir de banques d'acides nucléiques, en utilisant comme sonde la séquence native ou un fragment de celle-ci.

Ces dérivés sont notamment des molécules ayant une plus grande affinité pour leurs sites de fixation, des séquences permettant une expression améliorée in vivo, des molécules présentant une plus grande résistance aux protéases, des molécules ayant une efficacité thérapeutique plus grande ou des effets secondaires moindres, ou éventuellement de nouvelles propriétés biologiques.
Parmi les dérivés préférés, on peut citer plus particulièrement les variants naturels, les molécules dans lesquelles un ou plusieurs résidus ont été substitués, les dérivés obtenus par délétion de régions n'intervenant pas ou peu dans l'interaction avec les sites de liaison considérés ou exprimant une activité indésirable, et les dérivés comportant par rapport à la séquence native des résidus supplémentaires, tels que par exemple un signal de sécrétion et/ou un peptide de jonction.
Selon un mode de réalisation privilégié de l'invention, la séquence d'ADN, codant pour le GDNF ou l'un de ses dérivés, intègre également un signal de sécrétion permettant de diriger le GDNF synthétisé dans les voies de sécrétion des cellules infectées. Selon un mode préféré de l'invention, la séquence d'ADN contient en position 5' et en phase de lecture de la séquence codante du GDNF, une séquence de secrétion. De cette manière, le GDNF synthétisé est avantageusement libéré dans les compartiments extracellulaires et peut ainsi activer ses récepteurs. Le signal de sécrétion est avantageusement le propre signal du GDNF (désigné ci-après sous l'appellation "pré"). Toutefois, il peut également s'agir d'un signal de sécrétion hétérologue ou même artificiel. Avantageusement la séquence d'ADN code pour le pre-GDNF et plus particulièrement le pre-GDNF humain.
Avantageusement, la séquence codant pour le GDNF est placée sous le contrôle de signaux permettant son expression dans les cellules nerveuses. Préférentiellement, il s'agit de signaux d'expression hétérologues, c'est-à-dire de signaux différents de ceux naturellement responsables de l'expression du GDNF. Il peut s'agir en particulier de séquences responsables de l'expression d'autres protéines, ou de séquences synthétiques. Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs E1A, MLP, CMV, LTR-RSV, etc. En outre, ces séquences d'expression peuvent être modifiées par addition de séquences d'activation, de régulation, ou permettant une expression tissu-spécifique. Il peut en effet être particulièrement intéressant d'utiliser des signaux d'expression actifs spécifiquement ou majoritairement dans les cellules nerveuses, de manière à ce que la séquence d'ADN ne soit exprimée et ne produise son effet que lorsque le virus a effectivement infecté une cellule nerveuse. A cet égard, on peut citer par exemple les promoteurs de l'énolase neurone-spécifique, de la GFAP, etc.
Dans un premier mode de réalisation particulier, l'invention concerne l'utilisation d'un adénovirus recombinant défectif comprenant une séquence d'ADNc codant pour le preGDNF humain sous le contrôle du promoteur LTR-RSV.
Dans un autre mode de réalisation particulier, l'invention concerne l'utilisation d'un adénovirus recombinant défectif comprenant une séquence d'ADNg codant pour le preGDNF humain sous le contrôle du promoteur LTR-RSV.
La demanderesse a en effet montré que le promoteur LTR du virus du sarcome de rous (RSV) permettait une expression durable et importante du GDNF dans les cellules du système nerveux, notamment central.
Toujours dans un mode préféré, l'invention concerne l'utilisation d'un adénovirus recombinant défectif comprenant une séquence d'ADN codant pour tout ou une partie active du GDNF humain ou d'un dérivé de celui-ci sous le contrôle d'un promoteur permettant une expression majoritaire dans le système nerveux.
Un mode particulièrement préféré de mise en oeuvre de la présente invention réside dans l'utilisation d'un adenovirus recombinant défectif comprenant les séquences ITR, une séquence permettant l'encapsidation, une séquence d'ADN codant pour le facteur neurotrophique humain dérivé des cellules Gliales (hGDNF) ou un dérivé de celui-ci sous le contrôle d'un promoteur permettant une expression majoritaire dans le système nerveux, et dans lequel le gène E1 et au moins un des gènes E2, E4, L1-L5 est non fonctionnel.
Les adénovirus défectifs utilisés dans l'invention sont des adénovirus incapables de se répliquer de façon autonome dans la cellule cible. Généralement, le génome des adénovirus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par la séquence d'ADN codant pour le GDNF.
Préférentiellement, le virus défectif utilisé dans l'invention conserve les séquences de son génome qui sont nécessaires à l'encapsidation des particules virales. Encore plus préférentiellement, comme indiqué ci-avant, le génome du virus recombinant défectif selon l'invention comprend les séquences ITR, une séquence permettant l'encapsidation, le gène E1 non fonctionnel et au moins un des gènes E2, E4, L1-L5 non fonctionnel.

Il existe différents sérotypes d'adénovirus, .dont la structure et les propriétés varient quelque peu. Parmi ces sérotypes, on préfère utiliser dans le cadre de la présente invention les adénovirus humains de type 2 ou 5 (Ad 2 ou Ad 5) ou les adénovirus d'origine animale (voir demande FR 93 05954). Parmi les adénovirus d'origine animale utilisables dans le cadre de la présente invention on peut citer les adénovirus d'origine canine, bovine, murine, (exemple : Mav1, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise dans le cadre de l'invention des adénovirus d'origine humaine ou canine ou mixte.

Les adénovirus recombinants défectifs utilisés dans l'invention peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide portant entre autre la séquence d'ADN codant pour le GDNF. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %). Des stratégies de construction de vecteurs dérivés des adénovirus ont également été décrites dans les demandes n° FR 93 05954 et FR 93 08596 qui sont incorporées à la présente par référence.
Ensuite, les adénovirus qui se sont multipliés sont récupérés et purifiés selon les techniques classiques de biologie moléculaire, comme illustré dans les exemples.

Les propriétés particulièrement avantageuses des vecteurs de l'invention découlent notamment de la construction utilisée (adénovirus défectif, délété de certaines régions virales), du promoteur utilisé pour l'expression de la séquence codant pour le GDNF (promoteur viral ou tissu-spécifique de préférence), et des méthodes d'aministration dudit vecteur, permettant l'expression efficace et dans les tissus appropriés du GDNF. La présente invention fournit ainsi des vecteurs viraux utilisables directement en thérapie génique, particulièrement adaptés et efficaces pour diriger l'expression du GDNF in vivo. La présente invention offre ainsi une nouvelle approche particulièrement avantageuse pour le traitement et/ou la prévention de l'ALS.

La présente invention décrit également une composition pharmaceutique comprenant un ou plusieurs adénovirus recombinants défectifs tels que décrits précédemment. Ces compositions pharmaceutiques peuvent être formulées en vue d'administrations par voie topique, orale, parentérale, intranasale, intraveineuse, intramusculaire, sous-cutanée, intraoculaire, transdermique, etc. De préférence, les compositions pharmaceutiques de l'invention contiennent un véhicule pharmaceutiquement acceptable pour une formulation injectable, notamment pour une injection directe dans le système nerveux du patient. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. L'injection directe dans le système nerveux du patient est avantageuse car elle permet de concentrer l'effet thérapeutique au niveau des tissus affectés. L'injection directe dans le système nerveux central du patient est avantageusement réalisée au moyen d'un appareil d'injection stéréotaxique. L'emploi d'un tel appareil permet en effet de cibler avec une grande précision le site d'injection.

A cet égard, l'invention décrit également une méthode de traitement de l'ALS comprenant l'administration à un patient d'un adénovirus recombinant tel que défini ci-avant. Plus particulièrement, l'invention décrit une méthode de traitement de l'ALS comprenant l'administration stéréotaxique d'un adénovirus recombinant tel que défini ci-avant.

Les doses d'adénovirus recombinant défectif utilisées pour l'injection peuvent être adaptées en fonction de différents paramètres, et notamment en fonction du mode d'administration utilisé, de la pathologie concernée ou encore de la durée du traitement recherchée. D'une manière générale, les adénovirus recombinants utilisés dans l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, puis mesure, généralement après 48 heures, du nombre de plages de cellules infectées. Les techniques de détermination du titre pfu d'une solution virale sont bien documentées dans la littérature.

Un autre objet de l'invention concerne l'utilisation d'une cellule de mammifère infectée par un ou plusieurs adénovirus recombinants défectifs tels que décrits ci-dessus pour la préparation d'une composition pharmaceutique destinée au traitement et/ou à la prévention de l'ALS. Il peut s'agir en particulier de fibroblastes, myoblastes, hépatocytes, kératinocytes, cellules endothéliales, cellules Gliales, etc.
Les cellules peuvent être issues de cultures primaires. Celles-ci peuvent être prélevées par toute technique connue de l'homme du métier, puis mises en culture dans des conditions permettant leur prolifération. S'agissant plus particulièrement de fibroblastes, ceux-ci peuvent être aisément obtenus à partir de biopsies, par exemple selon la technique décrite par Ham [Methods Cell.Biol. 21a (1980) 255]. Ces cellules peuvent être utilisées directement pour l'infection par les adénovirus, ou conservées, par exemple par congélation, pour l'établissement de banques autologues, en vue d'une utilisation ultérieure. Les cellules selon l'invention peuvent également être des cultures secondaires, obtenues par exemple à partir de banques préétablies.
Les cellules en culture sont ensuite infectées par des adénovirus recombinants, pour leur conférer la capacité de produire du GDNF. L'infection est réalisée in vitro selon des techniques connues de l'homme du métier. En particulier, selon le type de cellules utilisé et le nombre de copies de virus par cellule désiré, l'homme du métier peut adapter la multiplicité d'infection et éventuellement le nombre de cycles d'infection réalisé. Il est bien entendu que ces étapes doivent être effectuées dans des conditions de stérilité appropriées lorsque les cellules sont destinées à une administration in vivo. Les doses d'adénovirus recombinant utilisées pour l'infection des cellules peuvent être adaptées par l'homme du métier selon le but recherché. Les conditions décrites ci-avant pour l'administration in vivo peuvent être appliquées à l'infection in vitro.

Un autre objet de l'invention concerne l'utilisation de cellules mammifère infectées par un ou plusieurs adénovirus recombinants défectifs telles que décrites ci-dessus, et une matrice extracellulaire. Préférentiellement, les implants selon l'invention comprennent 10⁵ à 10¹⁰ cellules. Plus préférentiellement, ils en comprennent 10⁶ à 10⁸.

Plus particulièrement, dans les implants utilisés dans l'invention, la matrice extracellulaire comprend un composé gélifiant et éventuellement un support permettant l'ancrage des cellules.
Pour la préparation des implants, différents types de gélifiants peuvent être employés. Les gélifiants sont utilisés pour l'inclusion des cellules dans une matrice ayant la constitution d'un gel, et pour favoriser l'ancrage des cellules sur le support, le cas échéant Différents agents d'adhésion cellulaire peuvent donc être utilisés comme gélifiants, tels que notamment le collagène, la gélatine, les glycosaminoglycans, la fibronectine, les lectines, etc. De préférence, on utilise dans le cadre de la présente invention du collagène. Il peut s'agir de collagène d'origine humaine, bovine ou murine. Plus préférenciellement, on utilise du collagène de type L
Comme indiqué ci-avant, les compositions ulilisées dans l'invention comprennent avantageusement un support permettant l'ancrage des cellules. Le terme ancrage désigne toute forme d'interaction biologique et/ou chimique et/ou physique entraînant l'adhésion et/ou la fixation des cellules sur le support. Par ailleurs, les cellules peuvent soit recouvrir le support utilisé, soit pénétrer à l'intérieur de ce support, soit les deux. On préfère utiliser dans le cadre de l'invention un support solide, non toxique et/ou bio-compatible. En particulier, on peut utiliser des fibres de polytétrafluoroéthylène (PTFE) ou un support d'origine biologique.
Les implants peuvent être implantés en différents sites de l'organisme. En particulier, l'implantation peut être effectuée au niveau de la cavité péritonéale, dans le tissu sous-cutané (région sus-pubienne, fosses iliaques ou inguinales, etc), dans un organe, un muscle, une tumeur, le système nerveux central, ou encore sous une muqueuse. Les implants sont particulièrement avantageux en ce sens qu'ils permettent de contrôler la libération du produit thérapeutique dans l'organisme : Celle-ci est tout d'abord déterminée par la multiplicité d'infection et par le nombre de cellules implantées. Ensuite, la libération peut être contrôlée soit par le retrait de l'implant, ce qui arrête définitivement le traitement, soit par l'utilisation de systèmes d'expression régulable, permettant d'induire ou de réprimer l'expression des gènes thérapeutiques.

La présente invention offre ainsi un moyen très efficace pour le traitement ou la prévention de l'ALS. Les vecteurs adénoviraux présentent en outre des avantages importants, liés notamment à leur très haute efficacité d'infection des cellules nerveuses, permettant de réaliser des infections à partir de faibles volumes de suspension virale. De plus, l'infection par les adénovirus est très localisée au site d'injection, ce qui évite les risques de diffusion aux structures cérébrales voisines.
En outre, ce traitement peut concerner aussi bien l'homme que tout animal tel que les ovins, les bovins, les animaux domestiques (chiens, chats, etc), les chevaux, les poissons, etc.
La présente invention sera plus complètement décrite à l'aide des exemples qui suivent, qui doivent être considérés comme illustratifs et non limitatifs.

### Légende de la figure

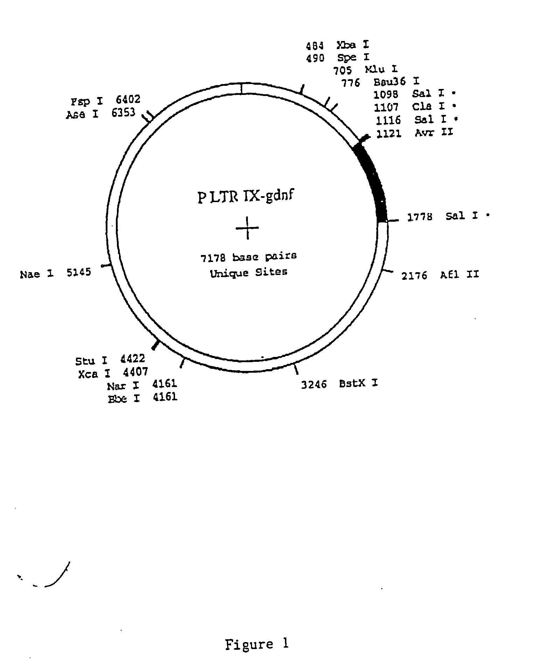

### Figure 1 :Représentation du vecteur pLTR IX-GDNF

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].
Les plasmides de type pBR322, pUC et les phages de la série M13 sont d'origine commerciale (Bethesda Research Laboratories).
Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.
Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.
La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.
L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.
La vérification des séquences nucléotidiques peut être effectuée par la méthode développée par Sanger et aL [Proc. Natl. Acad. Sci. USA, 74 (1977) 5463-5467] en utilisant le kit distribué par Amersham.

### Exemples

### Exemple 1 : Construction du vecteur pLTR IX-GDNF.

Cet exemple décrit la construction du vecteur pLTR IX-GDNF contenant la séquence codant pour le pre-GDNF de rat sous contrôle du LTR du virus RSV, ainsi que des séquences de l'adénovirus permettant la recombinaison in vivo.

Clonage d'un ADNc codant pour le pre-GDNF de rat. Le clonage a lieu par la technique PCR à partir d'ADNc de cellules gliales de rat obtenu par reverse transcription d'ARN provenant de ces cellules, en utilisant comme amorce les oligonucléotides suivants :
Oligonucléotide 5' : CCGTCGACCTAGGCCACCATGAAGTTATGGGATGTC
Oligonucléotide 3' : CCGTCGACATGCATGAGCTCAGATACATCCACACC
Les fragments obtenus par la technique de PCR, purifiés sur gel, coupés par l'enzyme de restriction Sall ont été insérés dans un plamide Buescript (Stratagène), dans le site Sall. Une séquence de polyadénylation provenant de SV40 avait été introduite auparavant dans le site Xho1 du même plasmide. Ce plasmide a pour nom SK-GDNF-PolyA.
Le vecteur pLTRIX-GDNF a été obtenu en introduisant entre les sites ClaI et EcoRV du palsmide pLTRIX (Stratford, Perricaudet et al J; Clin. Invest 90(1992) p626) un insert obtenu par coupure de SK-GDNF-PolyA par ClaI et KpnI (extrémités KpnI rendues franches)

### Exemple 2. Construction des adénovirus recombinants contenant une séquence codant pour le GDNF

Le vecteur pLTR IX-GDNF a été linéarisé et cotransfecté avec un vecteur adénoviral déficient, dans les cellules helper (lignée 293) apportant en trans les fonctions codées par les régions E1 (E1A et E1B) d'adénovirus.
Plus précisément, l'adénovirus Ad-GDNF a été obtenu par recombinaison homologue in vivo entre l'adénovirus mutant Ad-dl1324 (Thimmappaya et al., Cell 31 (1982) 543) et le vecteur pLTR IX-GDNF, selon le protocole suivant : le plasmide pLTR IX-GDNF et l'adénovirus Ad-dl1324, linéarisé par l'enzyme ClaI, ont été co-transfectés dans la lignée 293 en présence de phosphate de calcium, pour permettre la recombinaison homologue. Les adénovirus recombinants ainsi générés ont été sélectionnés par purification sur plaque. Après isolement, l'ADN de l'adénovirus recombinant a été amplifié dans la lignée cellulaire 293, ce qui conduit à un surnageant de culture contenant l'adénovirus défectif recombinant non purifié ayant un titre d'environ 10¹⁰ pfu/ml.

Les particules virales sont ensuite purifiées par centrifugation sur gradient

### Exemple 3: Transfert in vivo du gène GDNF par un adénovirus recombinant à des rats présentant une lésion de la voie nigro-striée.

Cet exemple décrit le transfert du gène GDNF in vivo au moyen d'un vecteur adénoviral selon l'invention. Il montre sur un modèle animal de la lésion de la voie nigro-striée, que les vecteurs de l'invention permettent d'induire l'expression in vivo de quantités thérapeutiques de GDNF.
Sur des rats préalablement anesthésiés, la voie nigro-striée a été lésée au niveau du faisceau mésencéphalique médian (MFB) par injection de la toxine 6-hydroxy dopamine (6OH-DA). Cette lésion chimique par injection a été unilatérale, suivant les coordonnées stéréotaxiques suivantes : AP : 0 et -1; ML : +1,6; V : -8,6 et -9 (les coordonnées AP et ML sont déterminées par rapport au bregma, la coordonnée V par rapport à la dure-mère). La barre d'incisive est fixée au niveau +5 mm.

L'adénovirus recombinant GDNF a été injecté immédiatement après la lésion, dans la substance noire et le striatum, du coté de la lésion. Plus particulièrement, l'adénovirus injecté est l'adénovirus Ad-GDNF préparé précédemment, utilisé sous forme purifiée (3,5 10⁶ pfu/µl), dans une solution saline phosphate (PBS).

Les injections ont été réalisées à l'aide d'une canule (diamètre extérieur 280 µm) connectée à une pompe. La vitesse d'injection est fixée à 0,5 µl/min, après quoi, la canule reste en place pendant 4 minutes supplémentaires avant d'être remontée. Les volumes d'injection dans le striatam et la substance noire sont respectivement 2x3 µl et 2 µl. La concentration d'adénovirus injectée est de 3,5. 10⁶ pfu/µl.

Pour l'injection dans la substance noire, les coordonnées stéréotaxiques sont les suivantes : AP=-5,8; ML=+2; V=-7,5 (les coordonnées AP et ML sont déterminées par rapport au bregma, la coordonnée V par rapport à la dure-mère).

Pour les injections dans le striatum, les coordonnées stéréotaxiques sont les suivantes : AP=+0,5 et -0,5; ML=3; V=-5,5 (les coordonnées AP et ML sont déterminées par rapport au bregma, la coordonnée V par rapport à la dure-mère).

Les effets thérapeutiques de l'administration de l'adénovirus selon l'invention ont été mis en évidence par trois types d'analyse : une analyse histologique et immunohistochimique, une analyse quantitative et une analyse comportementale.

### Analyse histologique et immunohistochimique

La lésion chimique de la voie nigro-striée induit une perte neuronale dans la substance noire ainsi que la dénervation dopaminergique dans le striatum (révélées en immunohistologie par un anticorps anti-tyrosine hydroxylase, TH).
L'analyse histologique des cerveaux injectés est réalisée 3 semaines après l'injection intracérébrale de l'adénovirus Ad-GDNF dans les conditions décrites dans l'exemple 6. Les coupes sériées coronales de 30 µm d'épaisseur sont réalisées dans la substance noire et le striatum. Des coupes espacées de 180 µm (1 coupe sur 6) sont colorées au crésyl violet (pour évaluer la densité neuronale) et immunomarquées par un anticorps anti tyrosine hydroxylase (TH) (pour détecter les neurones dopaminergiques dans la substance noire et leur innervation dans le striatum).

### Analyse quantitative

Le nombre de neurones dopaminergiques (TH positifs), dans la substance noire est le paramètre d'évaluation des effets de l'adénovirus Ad-GDNF. Le dénombrement est réalisé sur un échantillon (1 coupe sur 6 sur toute la longueur de la substance noire). Pour chaque coupe, les neurones TH positifs sont dénombrés séparément des 2 cotés de la substance noire. Les résultats cumulés pour toutes les coupes sont exprimés en proportion : nombre de neurones TH positifs du coté lésé par rapport au nombre de neurones TH positifs du coté non lésé.

### Analyse comportementale

Afin d'évaluer les effets fonctionnels protecteurs d'une injection d'adénovirus Ad-GDNF sur la lésion de la voie nigro-striée, les performances sensori-motrices des animaux sont analysées au cours de 2 tests comportementaux : le test de la rotation induite par des agonistes dopaminergiques (apomorphine, amphétamine et lévodopa), et le test de préhension ("paw-reaching").

## Revendications

1. Utilisation d'un adénovirus recombinant défectif dépourvu des régions de son génome qui sont nécessaires à sa réplication autonome dans une cellule cible et comprenant au moins une séquence d'ADN codant pour tout ou une partie active du GDNF ou d'un de ses placée sous le contrôle de signaux permettant son expression dans les cellules nerveuses pour la préparation d'une composition pharmaceutique destinée au traitement et/ou à la prévention de l'ALS.

2. Utilisation selon la revendication 1 **caractérisée en ce que** la séquence d'ADN contient en position 5' et en phase de lecture de la séquence codante du GDNF, une séquence de sécrétion.

3. Utilisation selon la revendication 1 ou 2 **caractérisée en ce que** la séquence d'ADN est une séquence d'ADNc.

4. Utilisation selon la revendication 1 ou 2 **caractérisée en ce que** la séquence d'ADN est une séquence d'ADNg

5. Utilisation selon l'une des revendications 1 à 4 **caractérisée en ce que** la séquence d'ADN code pour le GDNF humain.

6. Utilisation selon l'une des revendications 1 à 5 **caractérisée en ce que** les signaux d'expression sont choisis parmi les promoteurs viraux, de préférence parmi les promoteurs E1A, MLP, CMV et LTR-RSV.

7. Utilisation selon la revendication 1 **caractérisée en ce que** l'adénovirus comprend une séquence d'ADNc codant pour le pré-GDNF humain sous le contrôle du promoteur LTR-RSV.

8. Utilisation selon la revendication 1 **caractérisée en ce que** l'adénovirus comprend une séquence d'ADNg codant pour le pré-GDNF humain sous le contrôle du promoteur LTR-RSV.

9. Utilisation selon la revendication 1 **caractérisée en ce que** l'adénovirus comprend une séquence d'ADN codant pour tout ou une partie active du facteur neurotrophique humain dérivé des cellules Gliales (hGDNF) ou d'un dérivé de celui-ci sous le contrôle d'un promoteur permettant une expression majoritaire dans les cellules nerveuses.

10. Utilisation selon la revendication 9 **caractérisée en ce que** le promoteur est choisi parmi le promoteur de l'énolase neurone spécifique et le promoteur de la GFAP.

11. Utilisation selon l'une des revendications 1 à 10 **caractérisée en ce que** l'adénovirus comprend les ITR et une séquence permettant l'encapsidation, et dans lequel le gène E1 et au moins un des gènes E2, E4, L1-L5 sont non fonctionnels.

12. Utilisation selon l'une des revendications 1 à 11 **caractérisée en ce qu'**il sagit d'un adénovirus humain de type Ad 2 ou Ad 5 ou canin de type CAV-2.

13. Utilisation d'une cellule de mammifère infectée par un ou plusieurs adénovirus recombinants défectifs dépourvu des régions de son génome qui sont nécessaires à sa réplication autonome dans une cellule cible et comprenant au moins une séquence d'ADN codant pour tout ou une partie active du GDNF ou d'un de ses dérivés placée sous le contrôle de signaux permettant son expression dans les cellules nerveuses pour la préparation d'une composition pharmaceutique destinée au traitement et/ou à la prévention de l'ALS.

14. Utilisation selon la revendication 13 **caractérisée en ce qu'**il s'agit d'une cellule humaine.

15. Utilisation selon la revendication 13 **caractérisée en ce qu'**il s'agit d'une cellule humaine de type fibroblaste, myoblaste, hépatocyte, cellule endothéliale. cellule Gliales ou kératinocyte.

16. Utilisation selon l'une des revendications 13 à 15 **caractérisée en ce que** les cellules sont utilisées avec une matrice extracellulaire.

17. Utilisation selon la revendications 16 **caractérisée en ce que** la matrice extracellulaire comprend un composé gélifiant choisi de préférence parmi le collagène, la gélatine, les glucosaminoglyans, la fibronectine et les lectines.

18. Utilisation selon les revendications 16 et 17 **caractérisée en ce que** la matrice extracellulaire comprend également un support permettant l'ancrage des cellules infectées.

19. Utilisation selon la revendication 18 **caractérisée en ce que** le support est constitué préférentiellement par des fibres de polytétratluoroétylène.

## Claims

1. Use of a defective recombinant adenovirus lacking the regions of its genome which are necessary for its autonomous replication in a target cell and comprising at least one DNA sequence encoding the whole, or an active part, of GDNF or a derivative thereof, under the control of signals enabling its expression in nerve cells for the preparation of a pharmaceutical composition intended to treat and/or prevent ALS.

2. Use according to claim 1, **characterized in that** the DNA sequence contains a secretory sequence in the 5' position and in reading frame with the sequence encoding GDNF.

3. Use according to claim 1 or 2, **characterized in that** the DNA sequence is a cDNA sequence.

4. Use according to claim 1 or 2, **characterized in that** the DNA sequence is a gDNA sequence.

5. Use according to any of claims 1 to 4, **characterized in that** the DNA sequence encodes the human GDNF.

6. Use according to any of claims 1 to 5, **characterized in that** the expression signals are chosen from viral promoters, preferably from the E1A, MLP, CMV and RSV LTR promoters.

7. Use according to claim 1, **characterized in that** the adenovirus comprises a cDNA sequence encoding human pre-GDNF under the control of the RSV LTR promoter.

8. Use according to claim 1, **characterized in that** the adenovirus comprises a gDNA sequence encoding the human pre-GDNF under the control of the RSV LTR promoter.

9. Use according to claim 1, **characterized in that** the adenovirus comprises a DNA sequence encoding the whole, or an active part, of the human glial cell-derived neurotrophic factor (hGDNF), or a derivative thereof, under the control of a promoter enabling a major expression in nerve cells.

10. Use according to claim 9, **characterized in that** the promoter is chosen from the promoter of the neurone-specific enolase and the GFAP promoter.

11. Use according to any of claims 1 to 10, **characterized in that** the adenovirus comprises the ITRs and a sequence enabling encapsidation, and in which the E1 gene and at least one of the genes E2, E4, L1-L5 is non-functional.

12. Use according to any of claims 1 to 11, **characterized in that** it is an Ad 2 or Ad 5 type human adenovirus or a CAV-2 type canine adenovirus.

13. Use of a mammalian cell infected with one or more defective recombinant adenoviruses lacking the regions of its genome which are necessary for its autonomous replication in a target cell and comprising at least one DNA sequence encoding the whole, or an active part, of GDNF or a derivative thereof, under the control of signals enabling its expression in nerve cells for the preparation of a pharmaceutical composition intended to treat and/or prevent ALS.

14. Use according to claim 13, **characterized in that** it is a human cell.

15. Use according to claim 13, **characterized in that** it is a human cell of the fibroblast, myoblast, hepatocyte, endothelial cell, glial cell or keratinocyte type.

16. Use according to any of claims 13 to 15, **characterized in that** cells are used with an extracellular matrix.

17. Use according to claim 16, **characterized in that** the extracellular matrix comprises a gel-forming compound preferably chosen from collagen, gelatin, glucoseaminoglycans, fibronectin and lectins.

18. Use according to claims 16 and 17, **characterized in that** the extracellular matrix further comprises a support for anchoring infected cells.

19. Use according to claim 18, **characterized in that** the support is preferably made of polytetrafluoroethylene fibres.

## Patentansprüche

1. Verwendung eines defekten rekombinanten Adenovirus, das ohne die Regionen seines Genoms ist, die für seine autonome Replikation in einer Zielzelle erforderlich sind, und wenigstens eine DNA-Sequenz umfasst, die für den gesamten oder einen aktiven Teil des GDNF oder eines seiner Derivate codiert und unter der Kontrolle von Signalen steht, die ihre Expression in den Nervenzellen erlauben, für die Zubereitung einer pharmazeutischen Zusammensetzung für die Behandlung und/oder Prävention der ALS.

2. Verwendung gemäß Anspruch 1, **dadurch gekennzeichnet, dass** die DNA-Sequenz in 5'-Position und im Leserahmen der codierenden Sequenz des GDNF eine Sekretionssequenz enthält.

3. Verwendung gemäß Anspruch 1 oder 2, **dadurch gekennzeichnet, dass** die DNA-Sequenz eine cDNA-Sequenz ist.

4. Verwendung gemäß Anspruch 1 oder 2, **dadurch gekennzeichnet, dass** die DNA-Sequenz eine gDNA-Sequenz ist.

5. Verwendung gemäß einem der Ansprüche 1 bis 4, **dadurch gekennzeichnet, dass** die DNA-Sequenz für den humanen GDNF codiert.

6. Verwendung gemäß einem der Ansprüche 1 bis 5, **dadurch gekennzeichnet, dass** die Expressionssignale aus den viralen Promotoren, vorzugsweise aus den E1A, MLP, CMV und LTR-RSV-Promotoren, ausgewählt sind.

7. Verwendung gemäß Anspruch 1, **dadurch gekennzeichnet, dass** das Adenovirus eine für den humanen Prä-GDNF codierende cDNA-Sequenz unter der Kontrolle des LTR-RSV-Promotors umfasst.

8. Verwendung gemäß Anspruch 1, **dadurch gekennzeichnet, dass** das Adenovirus eine für den humanen Prä-GDNF codierende gDNA-Sequenz unter der Kontrolle des LTR-RSV-Promotors umfasst.

9. Verwendung gemäß Anspruch 1, **dadurch gekennzeichnet, dass** das Adenovirus eine DNA-Sequenz, die für den gesamten oder einen aktiven Teil des von den Gliazellen stammenden humanen neurotrophen Faktors (hGDNF) oder eines Derivats davon codiert, unter der Kontrolle eines Promotors umfasst, der eine überwiegende Expression in den Nervenzellen erlaubt.

10. Verwendung gemäß Anspruch 9, **dadurch gekennzeichnet, dass** der Promotor aus dem Promotor der Neuronen-spezifischen Enolase und dem Promotor des GFAP ausgewählt ist.

11. Verwendung gemäß einem der Ansprüche 1 bis 10, **dadurch gekennzeichnet, dass** das Adenovirus die ITR und eine die Verpackung erlaubende Sequenz umfasst, und in dem das E1-Gen und wenigstens eines der E2, E4, L1-L5-Gene nicht funktionsfähig sind.

12. Verwendung gemäß einem der Ansprüche 1 bis 11, **dadurch gekennzeichnet, dass** es sich um ein humanes Adenovirus des Typs Ad 2 oder Ad 5 oder um ein canines Adenovirus des Typs CAV-2 handelt.

13. Verwendung einer Säugerzelle, die mit einem oder mehreren defekten rekombinanten Adenoviren infiziert ist, das ohne die Regionen seines Genoms ist, die für seine autonome Replikation in einer Zielzelle erforderlich sind, und wenigstens eine DNA-Sequenz umfasst, die für den gesamten oder einen aktiven Teil des GDNF oder eines seiner Derivate codiert und unter der Kontrolle von Signalen steht, die ihre Expression in den Nervenzellen erlauben, für die Zubereitung einer pharmazeutischen Zusammensetzung für die Behandlung und/oder Prävention der ALS.

14. Verwendung gemäß Anspruch 13, **dadurch gekennzeichnet, dass** es sich um eine humane Zelle handelt.

15. Verwendung gemäß Anspruch 13, **dadurch gekennzeichnet, dass** es sich um eine humane Zelle des Typs Fibroblast, Myoblast, Hepatozyt, Endothelzelle, Gliazelle oder Keratinozyt handelt.

16. Verwendung gemäß einem der Ansprüche 13 bis 15, **dadurch gekennzeichnet, dass** die Zellen mit einer extrazellulären Matrix verwendet sind.

17. Verwendung gemäß Anspruch 16, **dadurch gekennzeichnet, dass** die extrazelluläre Matrix einen Gelbildner umfasst, der vorzugsweise aus Kollagen, Gelatine, Glucosaminoglycanen, Fibronektin und Lektinen ausgewählt ist.

18. Verwendung gemäß Ansprüchen 16 und 17, **dadurch gekennzeichnet, dass** die extrazelluläre Matrix ebenfalls einen Träger umfasst, der die Verankerung der infizierten Zellen erlaubt.

19. Verwendung gemäß Anspruch 18, **dadurch gekennzeichnet, dass** der Träger vorzugsweise aus Polytetrafluorethylenfasern besteht.
